# EUROPEAN PATENT SPECIFICATION

(11) **EP 1 782 384 B1**
(45) Date of publication and mention of the grant of the patent: **02.07.2014**
(21) Application number: 05771390.1
(22) Date of filing: 11.07.2005
(51) Int. Cl.: G06T 7/00

(54) **SYSTEM AND METHOD FOR COLON WALL EXTRACTION IN THE PRESENCE OF TAGGED FECAL MATTER OR COLLAPSED COLON REGIONS**
SYSTEM UND VERFAHREN ZUR DARMWANDEXTRAKTION BEI ANWESENHEIT VON MARKIERTEN FÄKALIEN ODER KOLLABIERTEN DARMREGIONEN
SYSTEME ET PROCEDE D'EXTRACTION D'UNE PAROI DU COLON EN PRESENCE DE SELLES MARQUEES OU DE ZONES AFFAISSEES DU COLON

(30) Priority: 24.08.2004 US 604106 P; 07.07.2005 US 176533
(43) Date of publication of application: 09.05.2007
(73) Proprietor: Siemens Medical Solutions USA, Inc., Malvern, PA 19355-1406 (US)
(72) Inventor: JEREBKO, Anna, West Chester, Pennsylvania 19380 (US)
(74) Representative: Bruns, Olaf
(86) International application number: PCT/US2005/024412
(87) International publication number: WO 2006/023152

(56) References cited:
- US-A1- 2002 039 400
- US-B1- 6 556 696
- KRISHNAN S M ET AL: "Closed-boundary extraction of large intestinal lumen" ENGINEERING IN MEDICINE AND BIOLOGY SOCIETY, 1994. ENGINEERING ADVANCES: NEW OPPORTUNITIES FOR BIOMEDICAL ENGINEERS., PROCEEDINGS OF THE 16TH ANNUAL INTERNATIONAL CONFERENCE OF THE IEEE BALTIMORE, MD, USA 3-6 NOV. 1994, NEW YORK, NY, USA,IEEE, US, 3 November 1994 (1994-11-03), pages 610-611, XP010145416 ISBN: 0-7803-2050-6
- ZUCKER S W: "REGION GROWING: CHILDHOOD AND ADOLESCENCE", COMPUTER GRAPHICS AND IMAGE PROCESSING, ACADEMIC PRESS. NEW YORK, US, vol. 5, no. 3, 1 September 1976 (1976-09-01), pages 382-399, XP001149042, DOI: 10.1016/0146-664X(76)90027-7
- ANNA K. JEREBKO ET AL: "Multi network classification scheme for detection of colonic polyps in CT colonography data sets", PROC. SPIE, vol. 4683, 22 April 2002 (2002-04-22), pages 207-212, XP055052107,

## Description

### BACKGROUND OF THE INVENTION

### 1. Technical Field

The present invention relates to medical image analysis, and more particularly, to a system and method for extracting a colon wall in the presence of tagged fecal matter or collapsed colon regions.

### 2. Discussion of the Related Art

Colon cancer currently ranks as the second leading cause of cancer-related deaths in the world. Most colorectal cancers arise from initially adenomatous polyps. Studies have shown that early detection and removal of colonic polyps can reduce the risk of colon cancer, thus decreasing the mortality rate. Unfortunately, conventional methods for the detection of colonic polyps are invasive, uncomfortable and have associated morbidity.

Computed tomography (CT) colonography or virtual colonoscopy has emerged as a potential alternative screening method for colonic polyps as well as masses. It combines helical CT scanning of the abdomen with visualization tools from non-invasive assessment of the colonic mucosa. However, the interpretation of virtual colonoscopy exams is time-consuming and the accuracy of polyp detection may depend on the display techniques utilized and the level of physician expertise.

Methods of image segmentation are known. For example, US 2002/039400 discloses a method for generating a three-dimensional visualization image of an object such as an organ using volume visualization techniques and exploring the image using a guided navigation system which allows the operator to travel along a flight path and to adjust the view to a particular potion of the image of interest in order to identify abnormal features in the organ.

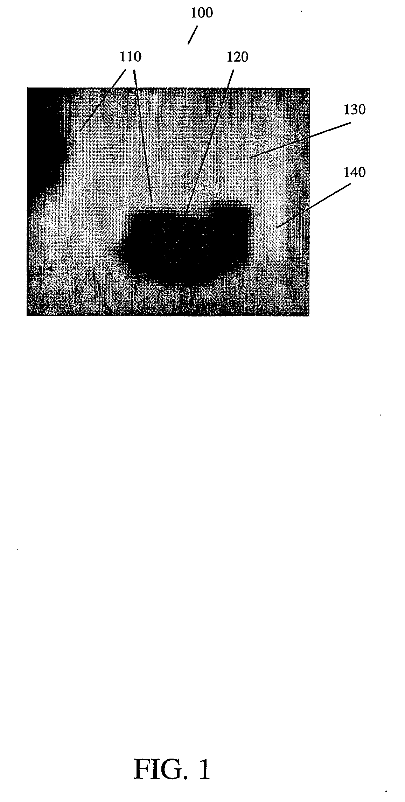

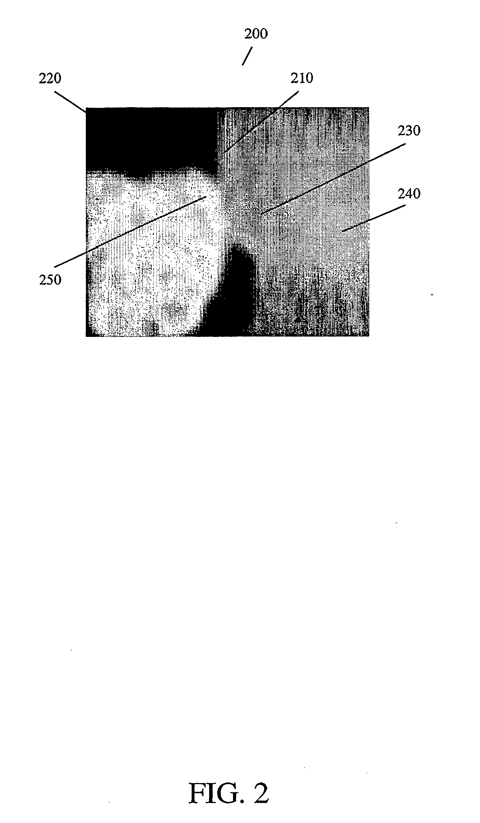

Recently, computer-aided diagnosis and detection (CAD) systems have been developed to automatically detect polyps and masses and provide the location of suspicious regions of the colon. Such CAD systems tend to employ algorithms for polyp detection that take into account the transition between a colon wall 110 and air 120 (e.g., the black area) as shown in FIG. 1. Further, in the presence of fecal matter or stool 250 in a colon 200 as shown in FIG. 2, the algorithms must deal with the removal of the fecal matter 250 because a colon wall 210 to air 220 (e.g., the black area) transition is not present in areas covered by the fecal matter 250.

The removal, however, of fecal matter tends to result in artifacts that change the look and properties of the surface of the colon wall and affect the subsequent analysis and detection of polyps by both a human observer and a CAD algorithm. Accordingly, there is a need for a technique capable of compensating for the presence of fecal matter in a colon so that its presence does not adversely affect the analysis and detection of polyps therein.

In addition, when collapsed colon regions are present, parts of the colon become virtually disconnected and it is difficult to trace the colon wall or its centerline in the collapsed regions. Accordingly, there is a need for a technique capable of analyzing a colon wall in the presence of collapsed regions, that improves the sensitivity of existing CAD algorithms, and that enhances the quality of virtual colonoscopy "fly-through" and centerline extraction techniques.

### SUMMARY OF THE INVENTION

The present invention overcomes the foregoing and other problems encountered in the known teachings by providing a system and method for extracting a colon wall in the presence of tagged fecal matter or collapsed colon regions that aids in the diagnosis and detection of diseases associated with the colon.

The invention is set out in the accompanying claims.

Additional features will become apparent in the following description, from the drawings and from the claims.

### BRIEF DESCRIPTION OF THE DRAWINGS

FIG. 1 is an image of a colon without fecal matter;
FIG. 2 is an image of a colon with tagged fecal matter;
FIG. 3 is a block diagram of a system for extracting a colon wall according to an exemplary embodiment of the present invention;
FIG. 4 is a flowchart illustrating a method for training a classifier to distinguish a colon wall from nearby objects according to an exemplary embodiment of the present invention;
FIG. 5 is a flowchart illustrating a method for extracting a colon wall according to an exemplary embodiment of the present invention; and
FIG. 6 is an image of a colon with non-uniformly tagged fecal matter.

### DETAILED DESCRIPTION OF EXEMPLARY EMBODIMENTS

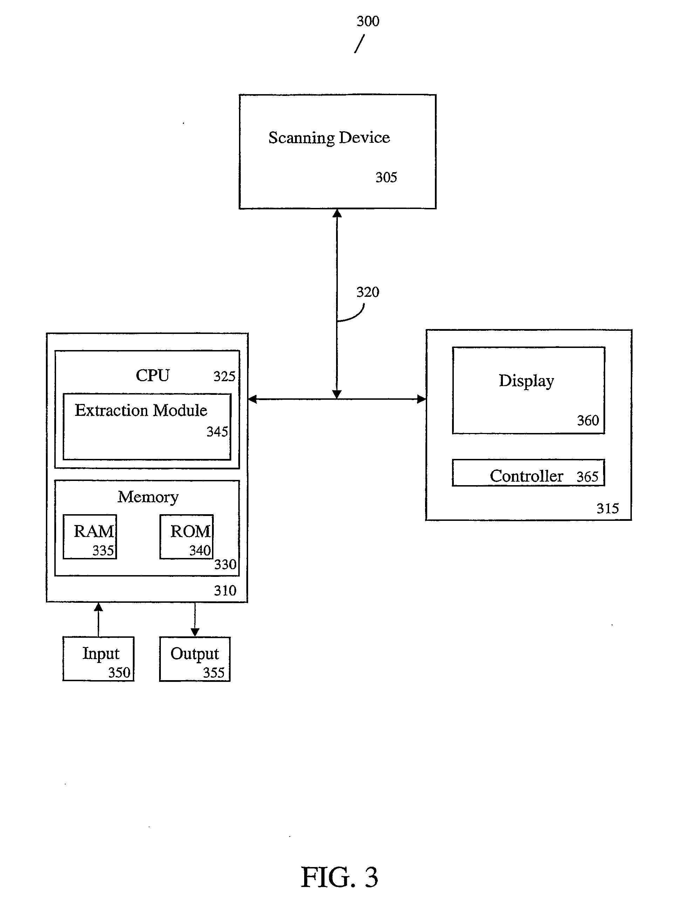

FIG. 3 is a block diagram of a system 300 for extracting a colon wall in the presence of tagged fecal matter or collapsed colon regions according to an exemplary embodiment of the present invention. As shown in FIG. 3, the system 300 includes, inter alia, a scanning device 305, a personal computer (PC) 310 and an operator's console 315 connected over, for example, an Ethernet network 320. The scanning device 305 may be an MR imaging device, a CT imaging device, a helical CT imaging device or a hybrid imaging device capable of CT, MR, positron emission tomography (PET) or other imaging techniques.

The PC 310, which may be a portable or laptop computer, a workstation, etc., includes a central processing unit (CPU) 325 and a memory 330, which are connected to an input 350 and an output 355. The CPU 325 includes an extraction module 345 that includes one or more methods for extracting a colon wall in the presence of tagged fecal matter or collapsed colon regions.

The memory 330 includes a random access memory (RAM) 335 and a read only memory (ROM) 340. The memory 330 can also include a database, disk drive, tape drive, etc., or a combination thereof. The RAM 335 functions as a data memory that stores data used during execution of a program in the CPU 325 and is used as a work area. The ROM 340 functions as a program memory for storing a program executed in the CPU 325. The input 350 is constituted by a keyboard, mouse, etc., and the output 355 is constituted by a liquid crystal display (LCD), cathode ray tube (CRT) display, or printer.

The operation of the system 300 is controlled from the operator's console 315, which includes a controller 365, for example, a keyboard, and a display 360, for example, a CRT display. The operator's console 315 communicates with the PC 310 and the scanning device 305 so that two-dimensional (2D) image data collected by the scanning device 305 can be rendered into three-dimensional (3D) data by the PC 310 and viewed on the display 360. It is to be understood that the PC 310 can be configured to operate and display information provided by the scanning device 305 absent the operator's console 315, using, for example, the input 350 and output 355 devices to execute certain tasks performed by the controller 365 and display 360.

The operator's console 315 may further include any suitable image rendering system/tool/application that can process digital image data of an acquired image dataset (or portion thereof) to generate and display 2D and/or 3D images on the display 360. More specifically, the image rendering system may be an application that provides 2D/3D rendering and visualization of medical image data, and which executes on a general purpose or specific computer workstation. Moreover, the image rendering system may enable a user to navigate through a 3D image or a plurality of 2D image slices. The PC 310 may also include an image rendering system/tool/application for processing digital image data of an acquired image dataset to generate and display 2D and/or 3D images.

As shown in FIG. 3, the extraction module 345 may also be used by the PC 310 to receive and process digital medical image data, which as noted above, may be in the form of raw image data, 2D reconstructed data (e.g., axial slices), or 3D reconstructed data such as volumetric image data or multiplanar reformats, or any combination of such formats. The data processing results can be output from the PC 310 via the network 320 to an image rendering system in the operator's console 315 for generating 2D and/or 3D renderings of image data in accordance with the data processing results, such as segmentation of organs or anatomical structures, color or intensity variations, and so forth.

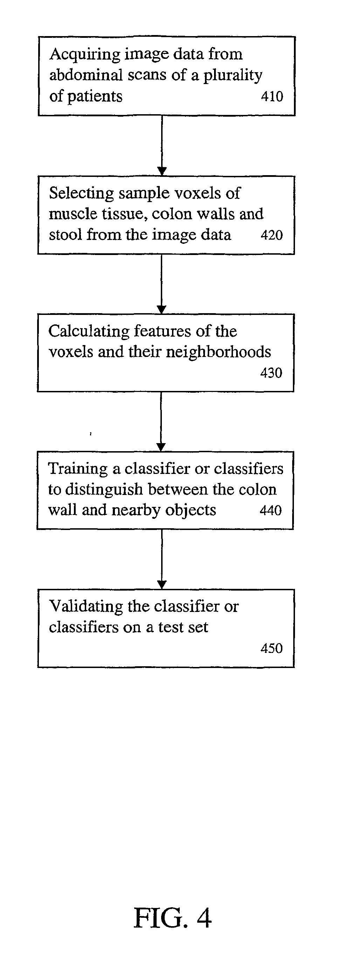

FIG. 4 illustrates a method for training a classifier to distinguish a colon wall from nearby objects. As shown in FIG. 4, image data from an abdominal scan or scans of a patient or patients is acquired (410). This is accomplished by using the scanning device 305, in this example a CT scanner, which is operated at the operator's console 315, to scan a patient's abdomen thereby generating a series of 2D image slices associated with a colon. The 2D image slices are then combined to form a 3D image.

After the CT image data is acquired, data samples of tagged and un-tagged fecal matter or stool, fat or muscle near the colon wall, the colon wall itself, collapsed colon regions, air, water or contrast matter are selected (420). Next, features such as statistical properties of voxels of individual sample points and the areas (e.g., neighborhoods) surrounding each of the voxels are calculated (430). The statistical properties that are calculated may be, for example, minimum, maximum and the moments of intensity (such as standard deviation, skewness and kurtosis). It is to be understood that the size of the neighborhoods could vary and can be determined based on a number of factors such as the thickness of the colon wall, collapsed colon regions, air, stool, fat or muscle being sampled.

Additional features characterizing shape, texture, distance, and statistical properties of local neighborhoods of different sizes around the sample points may be calculated in step 430. A wide variety of feature selection algorithms such as greedy search or genetic algorithms can be used to select relevant features and neighborhood sizes to be used in a subsequent classifier training technique.

Using the calculated features and statistical properties of the sample points and their local neighborhoods, a classifier or multiple classifiers are then trained to distinguish between the colon wall and nearby objects such as fat, muscle, air, stool or fluid inside the colon (440). It is to be understood that classifier training techniques employing semi-supervised, un-supervised of fully-supervised multi- or one-class classifications can be used in this step. Once the classifier or classifiers have been trained to distinguish between the colon wall and nearby objects, a validation such as a leave-one-out or N-fold cross-validation technique is performed, as well as a validation on an independent sequestered test set (450).

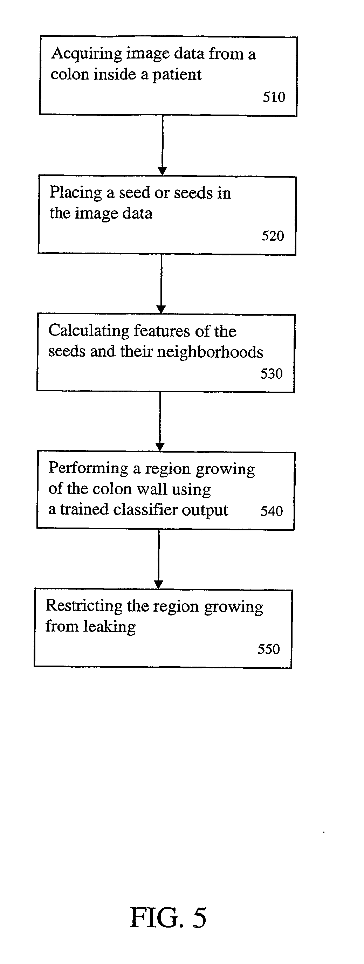

FIG. 5 is a flowchart showing an operation of a method for extracting a wall from a colon in the presence of tagged fecal matter or collapsed colon regions according to an exemplary embodiment of the present invention. As shown in FIG. 5, image data is acquired from, for example, an abdominal CT scan of a patient (510). This is accomplished by using the same or similar techniques described above with reference to step 410.

After the CT image data is acquired from the colon, a seed or seeds are placed in or around the colon (520). For example, the seeds may be placed in the colon wall 210 or in air pockets 220 inside the colon 200 as shown in FIG. 2. In addition, the seeds may be placed in the fat 240 or muscle 230, in the fecal matter 250 or in collapsed colon regions. The fecal matter 250 may be tagged by having a patient ingest an oral contrast agent such as barium or iodine that causes the fecal matter 250 or stool to have a distinctive coloring.

It is to be understood that in step 520 the seeds could be placed automatically by using an algorithm that determines the location of air pockets in a colon or stool in the colon and locates the colon wall. The seeds may also be placed manually. For example, a user may simply click on a desired seed point in or around a colon using a mouse cursor.

Once the seeds have been placed, features characterizing the shape, texture, distance and statistical properties of the seeds and local neighborhoods are calculated (530). This is accomplished by using the same or similar techniques described above with reference to step 430. The classifier or classifiers that were trained in step 440 are then applied together with a region growing of the colon wall 210 (540). More specifically, a region growing of the colon wall 210 is performed using the trained classifier or classifier's output and proximity and similarity measurements for all voxels. Upon completing the region growing, the connectivity of the colon wall 210 is determined, thus enabling the colon wall 210 to be extracted, traced or tracked. It is to be understood that in addition to applying the classifier or classifiers in the process of the region growing in step 540, other similarity measures may also be applied.

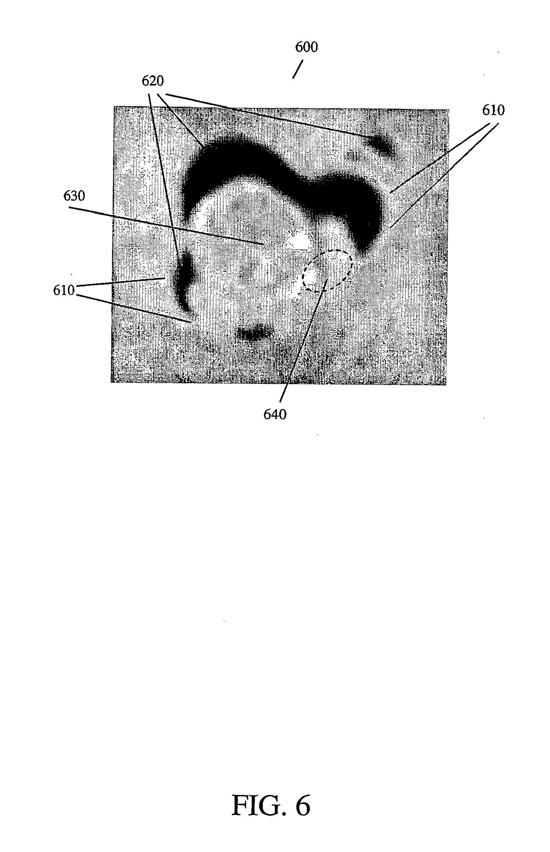

To further enhance the connectivity of a region grown colon wall and thus provide a medical practitioner or a polyp detection algorithm with a clearer and artifact free image of the colon wall for analysis, a set of post-processing steps may be performed on the region grown colon wall (550). One such process involves restricting the region growing from leaking into an un-tagged portion 640 of fecal matter 630 where there is no contrast agent 620. An example of this is shown in a colon image 600 of FIG. 6. For example, by removing voxels that have less than a predefined number of neighbors, applying morphological operations or by removing small detached clusters, a region growing can be prevented from leaking into the un-tagged portion 640 of fecal matter 630. Further, although care should be taken in regions where muscle 130 or fat 140 is close to the colon wall 110 as shown in FIG. 1, segmentation leakage into the muscle 130 does not affect the quality of the region growing inside the colon wall 110 that is of importance to clinicians, surface and volume rendering techniques and polyp detection algorithms in most instances.

In accordance with an exemplary embodiment of the present invention, a colon wall may be extracted as a thin muscle layer in the presence of tagged or partially tagged fecal matter or collapsed colon regions. Thus, enabling the inner portion of the colon wall to be visualized for a "fly-through" during a virtual colonoscopy, used for local endoscopic views of polyps located thereon or used in conjunction as an extension for or an alternative to manual or automated computer-aided diagnosis and polyp detection techniques.

It is to be further understood that because some of the constituent system components and method steps depicted in the accompanying figures may be implemented in software, the actual connections between the system components (or the process steps) may differ depending on the manner in which the present invention is programmed. Given the teachings of the present invention provided herein, one of ordinary skill in the art will be able to contemplate these and similar implementations or configurations of the present invention.

It should also be understood that the above description is only representative of illustrative embodiments. For the convenience of the reader, the above description has focused on a representative sample of possible embodiments, a sample that is illustrative of the principles of the invention. The description has not attempted to exhaustively enumerate all possible variations. That alternative embodiments may not have been presented for a specific portion of the invention, or that further undescribed alternatives may be available for a portion, is not to be considered a disclaimer of those alternate embodiments. Other applications and embodiments can be implemented without departing from the scope of the present invention as defined by the appended claims.

## Claims

1. A method for extracting a colon wall (210) from an image of a patient abdomen, comprising:
placing seeds (520) in the image;
determining (530) features of the seeds and voxels neighboring the seeds; and
performing (540) a region growing of the colon wall using the output of a classifier, the classifier trained to distinguish between the colon wall and nearby objects based on the features of the seeds and voxels neighboring the seeds,
wherein the features comprise minimum, maximum or moments of intensity, shape, or texture of the seeds and voxels neighboring the seeds, and wherein the nearby objects are fecal matter, air, muscle, fat or liquid.

2. The method of claim 1, wherein the seeds are placed in one of the colon wall, in air inside the colon, in fat near the colon wall, in fecal matter inside the colon or in a collapsed region of the colon wall.

3. The method of claim 2, wherein the seeds are placed automatically or manually.

4. The method of claim 2, wherein the fecal matter is tagged.

5. The method of claim 1, wherein the image of the patient abdomen is acquired using one of a CT or MR imaging technique.

6. The method of claim 1, further comprising:
acquiring image data of an abdominal scan of a patient;
selecting sample voxels from the image data;
determining features of the sample voxels and voxels neighboring the sample voxels;
training a classifier to distinguish between the colon wall and nearby objects; and
validating the classifier.

7. The method of claim 1, further comprising:
restricting the region growing from leaking.

8. A method according to Claim 1 for tracking a colon wall, comprising:
placing a plurality of seed voxels in the image, wherein the image is of a colon;
and
determining a connectivity of the colon wall by performing the region growing of the colon wall.

9. A method according to Claim 1 for locating polyps in a colon, comprising:
placing seeds in an image of a colon;
extracting a wall of the colon by performing the region growing of the colon wall; and
locating polyps on the colon wall using the extracted colon wall.

10. A system for extracting a colon wall (210) from an image of a patient abdomen, comprising:
a memory device for storing a program;
a processor in communication with the memory device, the processor operative with the program to:
place a seed (520) in the image;
determine (530) features of the seed and voxels neighboring the seed; and
perform (540) a region growing of the colon wall using the output of a classifier, the classifier trained to distinguish between the colon wall and nearby objects based on the features of the seed and voxels neighboring the seed,
wherein the features comprise minimum, maximum or moments of intensity, shape, or texture of the seeds and voxels neighboring the seeds, and wherein the nearby objects are fecal matter, air, muscle, fat or liquid.

11. The system of claim 11, wherein the seed is placed in one of the colon wall, in air inside the colon, in fat near the colon wall, in fecal matter or in a collapsed region of the colon wall.

12. The system of claim 11, wherein the processor is further operative with the program code to:
acquire image data of a patient abdomen;
select a sample voxel from the image data;
determine features of the sample voxel and voxels neighboring the sample voxel;
train a classifier to distinguish between the colon wall and nearby objects; and
validate the classifier.

13. The system of claim 11, wherein the processor is further operative with the program code to:
restrict the region growing from leaking.

14. The system of claim 11, wherein the image of the patient abdomen is acquired using one of a CT or MR imaging device.

## Patentansprüche

1. Verfahren zum Extrahieren einer Dickdarmwand (210) aus einem Bild eines Unterleibs eines Patienten, welches umfasst:
Platzieren von Seeds (520) in dem Bild;
Bestimmen (530) von Merkmalen der Seeds und von den Seeds benachbarten Voxeln; und
Durchführen (540) eines "Region Growing" der Dickdarmwand unter Verwendung des Ausgangs eines Klassifikators, wobei der Klassifikator dafür trainiert ist, zwischen der Dickdarmwand und in der Nähe befindlichen Objekten auf der Basis der Merkmale der Seeds und der den Seeds benachbarten Voxel zu unterscheiden,
wobei die Merkmale Minimum, Maximum oder Momente von Intensität, Form oder Textur der Seeds und der den Seeds benachbarten Voxel umfassen und wobei die in der Nähe befindlichen Objekte Fäkalien, Luft, Muskeln, Fett oder Flüssigkeit sind.

2. Verfahren nach Anspruch 1, wobei die Seeds entweder in der Dickdarmwand oder in Luft innerhalb des Dickdarms oder in Fett in der Nähe der Dickdarmwand oder in Fäkalien innerhalb des Dickdarms oder in einem kollabierten Bereich der Dickdarmwand platziert werden.

3. Verfahren nach Anspruch 2, wobei die Seeds automatisch oder manuell platziert werden.

4. Verfahren nach Anspruch 2, wobei die Fäkalien markiert werden.

5. Verfahren nach Anspruch 1, wobei das Bild des Unterleibs des Patienten unter Verwendung von entweder CT- oder MR-Bildgebungstechnik erfasst wird.

6. Verfahren nach Anspruch 1, welches ferner umfasst:
Erfassen von Bilddaten eines Unterleibsscans eines Patienten;
Auswählen von Probe-Voxeln aus den Bilddaten;
Bestimmen von Merkmalen der Probe-Voxel und von den Probe-Voxeln benachbarten Voxeln;
Trainieren eines Klassifikators dafür, zwischen der Dickdarmwand und in der Nähe befindlichen Objekten zu unterscheiden; und
Validieren des Klassifikators.

7. Verfahren nach Anspruch 1, welches ferner umfasst:
Verhindern einer Leckage des "Region Growing".

8. Verfahren nach Anspruch 1 zum Aufsuchen einer Dickdarmwand, welches umfasst:
Platzieren einer Vielzahl von Seed-Voxeln in dem Bild, wobei das Bild das eines Dickdarms ist;
und
Bestimmen einer Konnektivität der Dickdarmwand durch Durchführung des "Region Growing" der Dickdarmwand.

9. Verfahren nach Anspruch 1 zum Lokalisieren von Polypen in einem Dickdarm, welches umfasst:
Platzieren von Seeds in einem Bild eines Dickdarms;
Extrahieren einer Wand des Dickdarms durch Durchführung des "Region Growing" der Dickdarmwand; und
Lokalisieren von Polypen auf der Dickdarmwand unter Verwendung der extrahierten Dickdarmwand.

10. System zum Extrahieren einer Dickdarmwand (210) aus einem Bild eines Unterleibs eines Patienten, welches umfasst:
eine Speichereinrichtung zum Speichern eines Programms;
einen Prozessor, der mit der Speichereinrichtung in Kommunikation steht, wobei der Prozessor dafür ausgelegt ist, mit dem Programm:
ein Seed in dem Bild zu platzieren (520);
Merkmale des Seeds und von dem Seed benachbarten Voxeln zu bestimmen (530); und
ein "Region Growing" der Dickdarmwand unter Verwendung des Ausgangs eines Klassifikators durchzuführen (540), wobei der Klassifikator dafür trainiert ist, zwischen der Dickdarmwand und in der Nähe befindlichen Objekten auf der Basis der Merkmale des Seeds und der dem Seed benachbarten Voxel zu unterscheiden,
wobei die Merkmale Minimum, Maximum oder Momente von Intensität, Form oder Textur der Seeds und der den Seeds benachbarten Voxel umfassen und wobei die in der Nähe befindlichen Objekte Fäkalien, Luft, Muskeln, Fett oder Flüssigkeit sind.

11. System nach Anspruch 11, wobei das Seed entweder in der Dickdarmwand oder in Luft innerhalb des Dickdarms oder in Fett in der Nähe der Dickdarmwand oder in Fäkalien oder in einem kollabierten Bereich der Dickdarmwand platziert ist.

12. System nach Anspruch 11, wobei der Prozessor ferner dafür ausgelegt ist, mit dem Programmcode:
Bilddaten eines Unterleibsscans eines Patienten zu erfassen;
ein Probe-Voxel aus den Bilddaten auszuwählen;
Merkmale des Probe-Voxels und von dem Probe-Voxel benachbarten Voxeln zu bestimmen;
einen Klassifikator dafür zu trainieren, zwischen der Dickdarmwand und in der Nähe befindlichen Objekten zu unterscheiden; und
den Klassifikator zu validieren.

13. System nach Anspruch 11, wobei der Prozessor ferner dafür ausgelegt ist, mit dem Programmcode:
eine Leckage des "Region Growing" zu verhindern.

14. System nach Anspruch 11, wobei das Bild des Unterleibs des Patienten unter Verwendung entweder einer CT- oder einer MR-Bildgebungseinrichtung erfasst wird.

## Revendications

1. Procédé d'extraction d'une paroi colique (210) à partir d'une image de l'abdomen d'un patient, consistant :
à placer des graines (520) dans l'image ;
à déterminer (530) les caractéristiques des graines et des voxels avoisinant les graines, et
à réaliser (540) un accroissement de région de la paroi colique en utilisant la sortie d'un module de classification, le module de classification ayant appris à distinguer la paroi colique des objets voisins en se basant sur les caractéristiques des graines et des voxels avoisinant les graines,
étant entendu que les caractéristiques comprennent un minimum, un maximum ou des moments d'intensité, la forme ou la texture des graines et des voxels avoisinant les graines, et étant entendu que les objets voisins sont des matières fécales, de l'air, du muscle, de la graisse ou du liquide.

2. Procédé selon la revendication 1, dans lequel les graines sont placées dans la paroi colique, dans l'air contenu dans le côlon, dans la graisse avoisinant la paroi colique, dans les matières fécales contenues dans le côlon ou dans une zone affaissée de la paroi colique.

3. Procédé selon la revendication 2, dans lequel les graines sont placées automatiquement ou manuellement.

4. Procédé selon la revendication 2, dans lequel les matières fécales sont marquées.

5. Procédé selon la revendication 1, dans lequel on acquiert l'image de l'abdomen du patient en utilisant un procédé d'imagerie par tomographie assistée par ordinateur (CT) ou par résonance magnétique (MR).

6. Procédé selon la revendication 1, consistant par ailleurs :
à acquérir les données d'image d'un scanner abdominal d'un patient ;
à sélectionner des échantillons de voxels parmi les données d'image ;
à déterminer les caractéristiques des échantillons de voxels et de voxels avoisinant les échantillons de voxels ;
à apprendre à un module de classification à distinguer la paroi colique des objets voisins, et
à valider le module de classification.

7. Procédé selon la revendication 1, consistant par ailleurs :
à empêcher une fuite de l'accroissement de région.

8. Procédé selon la revendication 1 permettant de suivre une paroi colique, consistant :
à placer une pluralité de voxels de graines dans l'image, l'image étant celle d'un côlon, et
à déterminer des connexions de la paroi colique en réalisant l'accroissement de région de la paroi colique.

9. Procédé selon la revendication 1 permettant de localiser des polypes dans un côlon, consistant :
à placer des graines dans une image d'un côlon ;
à extraire une paroi du côlon en réalisant l'accroissement de région de la paroi colique, et
à localiser les polypes sur la paroi colique au moyen de la paroi colique extraite.

10. Système permettant d'extraire une paroi colique (210) à partir de l'image de l'abdomen d'un patient, comprenant :
un dispositif mémoire pour stocker un programme ;
un processeur en communication avec le dispositif mémoire, le processeur opérant avec le programme pour :
placer une graine (520) dans l'image ;
déterminer (530) des caractéristiques de la graine et des voxels avoisinant la graine, et
réaliser (540) un accroissement de région de la paroi colique au moyen de la sortie d'un module de classification, le module de classification ayant appris à distinguer la paroi colique des objets voisins en se basant sur les caractéristiques de la graine et des voxels avoisinant la graine,
étant entendu que les caractéristiques comprennent un minimum, un maximum ou des moments d'intensité, la forme ou la texture des graines et des voxels avoisinant les graines, et étant entendu que les objets voisins sont des matières fécales, de l'air, du muscle, de la graisse ou du liquide.

11. Système selon la revendication 11, dans lequel la graine est placée dans la paroi colique, dans l'air contenu dans le côlon, dans la graisse avoisinant la paroi colique, dans des matières fécales ou dans une zone affaissée de la paroi colique.

12. Système selon la revendication 11, dans lequel le processeur opère par ailleurs avec le code de programme pour :
acquérir des données d'image de l'abdomen d'un patient ;
sélectionner un échantillon de voxel parmi les données d'image ;
déterminer des caractéristiques de l'échantillon de voxel et des voxels avoisinant l'échantillon de voxel ;
apprendre à un module de classification à distinguer la paroi colique des objets voisins, et
valider le module de classification.

13. Système selon la revendication 11, dans lequel le processeur opère par ailleurs avec le code de programme pour :
empêcher une fuite de l'accroissement de région.

14. Système selon la revendication 11, dans lequel on acquiert l'image de l'abdomen du patient en utilisant un dispositif d'imagerie par CT ou par MR.
